(19) Europäisches Patentamt
European Patent Office
Office européen des brevets

(11) Veröffentlichungsnummer: **0 062 902**
**B1**

(12) # EUROPÄISCHE PATENTSCHRIFT

(45) Veröffentlichungstag der Patentschrift: **15.01.86**

(51) Int. Cl.⁴: **C 07 D 307/935**, C 07 C 177/00, A 61 K 31/34

(21) Anmeldenummer: **82103024.4**

(22) Anmeldetag: **08.04.82**

(54) **2,3,4-Trinor-m-inter-phenylen-prostaglandin-Derivate, Verfahren zu deren Herstellung sowie diese Verbindungen enthaltende pharmazeutische Präparate.**

(30) Priorität: **14.04.81 HU 96281**
**14.04.81 HU 96381**
**14.04.81 HU 96481**

(43) Veröffentlichungstag der Anmeldung: **20.10.82 Patentblatt 82/42**

(45) Bekanntmachung des Hinweises auf die Patenterteilung: **15.01.86 Patentblatt 86/3**

(84) Benannte Vertragsstaaten: **BE CH DE FR GB IT LI NL SE**

(56) Entgegenhaltungen:
**EP-A-0 045 842**
**DE-A-2 945 781**

**Die Akte enthält technische Angaben, die nach dem Eingang der Anmeldung eingereicht wurden und die nicht in dieser Patentschrift enthalten sind.**

(73) Patentinhaber: **CHINOIN Gyogyszer és Vegyészeti Termékek Gyára RT., To utca 1-5, H-1045 Budapest IV (HU)**

(72) Erfinder: **Székely, István, Dr., Krajcár u. 6, H-2120 Dunakeszi (HU)**
Erfinder: **Kékesi, Krisztina, Aprilis 4.- e u. 23, H-4029 Debrecen (HU)**
Erfinder: **Lovász geb. Gáspán, Mariann, Lakatos ut 28, H-1184 Budapest (HU)**
Erfinder: **Botár, Sándor, Baross u. 109, H-1082 Budapest (HU)**
Erfinder: **Hadházy, Pál, Dr., Baranyai u. 31/b, H-1117 Budapest (HU)**
Erfinder: **Rákoczi, István, Dr., Bombocz Z. u.16, H-1118 Budapest (HU)**
Erfinder: **Muszbek, László, Dr., Poroszlay u. 50, H-4032 Debrecen (HU)**
Erfinder: **Skopál, Judit, Szakasits Arpád u. 34/b, H-1115 Budapest (HU)**
Erfinder: **Stadler, István, Dr., Népstadion u. 18, H-1143 Budapest (HU)**
Erfinder: **Horváth, Károly, Dr., Szakasits Arpád u. 58/a, H-1115 Budapest (HU)**
Erfinder: **Kovács, Gábor, Dr., Róna park, 4, H-1142 Budapest (HU)**
Erfinder: **Körmöczy, Péter, Dr., Uri u. 33, H-1014 Budapest (HU)**

(74) Vertreter: **Lotterhos, Hans Walter, Dr.- Ing., Lichtensteinstrasse 3, D-6000 Frankfurt am Main 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 062 902 B1

LIBER, STOCKHOLM 1986

**Beschreibung**

Die Erfindung betrifft neue 2, 3, 4-Trinor-1, 5-inter-m-phenylen-prosta-cyclin-Derivate der allgemeinen Formel I

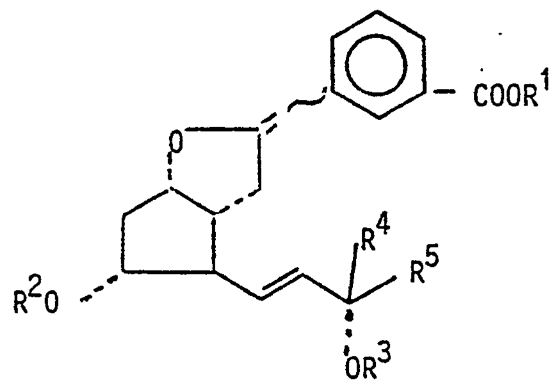

(I) ,

worin

$R^1$ Wasserstoff, $C_{1-4}$-Alkyl, Alkali- oder prim., sec., tert. oder quart. Ammoniumkation,

$R^2$ und $R^3$ unabhängig voneinander Wasserstoff, $C_{1-4}$-Alkanoyl, gegebenenfalls substituiertes Benzoyl oder Äthoxyäthyl,

$R^4$ Wasserstoff oder $C_{1-4}$-Alkyl und

$R^5$ n-Hexyl, n-Heptyl, Phenoxymethyl, m-Trifluormethyl-phenoxymethyl oder eine Gruppe der allgemeinen Formel

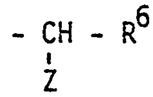

bedeuten, in der

Z Amino, gegebenenfalls durch Halogen substituiertes $C_{1-4}$-Alkanoylamino, Benzoylamino oder Tosylamino und

$R^6$ $C_{4-6}$-Alkyl, Phenyl oder Benzyl ist.

Die Verbindungen der allgemeinen Formel I sind Prostacyclin-Analoge mit verschiedenen pharmakologischen Wirkungen. Sie hemmen die Gerinnung der Blutplättchen, sie wirken desaggregierend auf Blutgerinnsel, sie haben eine gefäßerweiternde, eine cytoprotektive und eine ulcusheilende Wirkung.

Die Erfindung betrifft ferner ein Verfahren zur Herstellung dieser Verbindungen und diese Verbindungen enthaltender Arzneimittelpräparate.

Das Prostacyclin ($PGI_2$) ist eine endogene Substanz. Sie wurde 1976 von J.Vane u.a. endeckt (Nature 1976, 263, 663; Prostaglandins 1976, 12, 685 und 897), wobei festgestellt wurde, daß das $PGI_2$ in der haemostatischen Regulierung des Kreislaufsystems als antiaggregierendes und desaggregierendes Agens eine Rolle soielt.

Die chemische Synthese des Prostacyclins ist bereits mehreren Forschergruppen (z.B. Angewandte Chemie, International Edition 1978, 17, 293 ff., so wie die dort angeführten Referenzen) gelungen. Die praktische Anwendung des Prostacyclins - z.B. bei der Prophylaxe des Infarkts- wird jedoch durch dessen Instabilität, die auf die im Prostacyclinmolekül vorhandene exocyclische Enolätherkonfiguration zurückzuführen ist, stark eingeschränkt. Das Prostacyclin ist zwar in Form seines Natriumsalzes eine stabile und gut lagerbare Verbindung, aber unter physiologischen Bedingungen wird die aus dem Natriumsalz freigesetzte $PGI_2$-Säure schnell zu 6-Keto-$PGF_{1\alpha}$ hydrolysiert, das nicht die Wirkungen des $PGI_2$ aufweist. Es sind daher zahlreiche Untersuchungen gemacht worden, Prostacyclin-Analoge herzustellen, die chemisch stabil und in der Wirkung möglichst selektiver sind als $PGI_2$.

Aus der nicht vor veröffentlichten EP-A1 0 045 842 sind u.a. 2, 3, 4-Trinor-1, 5-inter-m-phenylen-prostacyclin-Derivate bekannt, die eine höhere Stabilität als $PGI_2$ aufweisen und die sich in der Kette am $C_{12}$-Atom vom Prostacyclin darin unterscheiden können, daß sie am $C_{15}$-Atom eine $CH_3$-Gruppe aufweisen und/oder am $C_{16}$-Atom zwei $CH_3$-Gruppen oder eine $C_2H_5$-Gruppe und/oder anstelle des $C_{16}$-$C_{20}$-Alkylrestes einen gegebenenfalls substituierten Cyclohexylrest enthalten.

Die 2, 3, 4-Trinor-1, 5-inter-m-phenylen-$PGI_2$-Derivate der Erfindung weisen ebenfalls eine wesentlich höhere chemische Stabilität als das Prostacyclin aus (selbst bei pH = 1 beträgt die Halbwertzeit des Abbaus einige Tage). Das wird auf die Wirkung der Phenylgruppe zurückgeführt, die sich zu dem Enoläther in Konjugation befindet, da das $\pi$ -Elektronensystem der Phenylgruppe durch die Konjugation die Reaktivität des Enoläthers vermindert. Außerdem wird durch die starre Struktur des Moleküls die bei dem Prostacyclin auftretende Hydrolyse verhindert, die durch die eigene Carboxylgruppe katalysiert wird.

Die wichtigste pharmakologische Wirkung der Verbindungen der Erfindung besteht darin, daß sie - ähnlich wie $PGI_2$ - die Aggregation der Blutplättchen hemmen und daß sie bereits gebildete Agglomerate auflösen können.

In einem Vergleichsversuch wurde die Hemmwirkung der Verbindungen der Erfindung und des $PGI_2$ auf ein an Blutplättchen reichem Plasma (PRP) von Mensch und Meerschweinchen gemessen. Die Messung erfolgte nach der Methode von Born. Die Aggregation wurde durch ADP ausgelöst, das in einer Konzentration von 4 µM/ml appliziert worden war. Bestimmt wurden die für eine 50 %ige und für eine 100 %ige Hemmung erforderliche Prostacyclin-Konzentration. Die Ergebnisse sind in der Tabelle I enthalten.

| Wirkstoff | $IC_{50}$ Human PRP | $IC_{100}$ Meerschw. PRP |
|---|---|---|
| $PGI_2$-Natriumsalz | 2 ng/ml | 1,75 ng/ml |
| 5(Z)-2,3,4-Trinor-1,5-inter-m-phenylen-20-methyl-$PGI_2$-Natriumsalz | 12,5 ng/ml | 14 ng/ml |

Die Verbindungen der Erfindung können in an sich bekannter Weisemit den in der pharmazeutischen Industrie üblichen Streck-, Füll- und Trägerstoffen zu Arzneimittelpräparaten formuliert werden. Diese können Teile der wirksamen Dosis oder deren ganzzahliges Vielfaches enthalten.

Als besonders vorteilhaft haben sich folgende Verbindungen erwiesen: 2, 3, 4, 17, 18, 19, 20-Heptanor-1,5-inter-m-phenylen-16-phenoxy-$PGI_2$-methylester, 2, 3, 4, 17, 18, 19, 20-Heptanor-1, 5-inter-m-phenylen-16-(3-trifluormethyl-phenoxy)-$PGI_2$-methylester, 2, 3, 4-Trinor-1, 5-inter-m-phenylen-16-amino-$PGI_2$-methylester, 2, 3, 4-Trinor-1, 5-inter-m-phenylen-16-acetamido-$PGI_2$-methylester, 2, 3, 4-Trinor-1, 5-inter-m-phenylen-16-benzoylamino-$PGI_2$-methylester, 2, 3, 4, 17, 18, 19, 20-Heptanor-1, 5-inter-m-phenylen-16-amino-$PGI_2$-methylester, 2, 3, 4, 18, 19, 20-Hexanor-1, 5-inter-m-phenylen-16-amino-17-phenyl-$PGI_2$-methylester, 5(Z)-2, 3, 4, 17, 18, 19, 20-Heptanor-1, 5-inter-m-phenylen-16-phenoxy-$PGI_2$-Natriumsalz, 5(E)-2, 3, 4, 17, 18, 19, 20-Heptanor-1, 5-inter-m-phenylen-16-phenoxy-$PGI_2$-Natriumsalz, 2, 3, 4, 17, 18, 19, 20-Heptanor-1, 5-inter-m-phenylen-16-(3-trifluormethyl-phenoxy)-$PGI_2$-Natriumsalz, 5(Z)-2, 3, 4-Trinor-1, 5-inter-m-phenylen-20-methyl-$PGI_2$-Natriumsalz, 5(E)-2, 3, 4-Trinor-1, 5-inter-m-phenylen-20-methyl-$PGI_2$-Natriumsalz, 2, 3, 4-Trinor-1, 5-inter-m-phenylen-20-äthyl-$PGI_2$-Natriumsalz, 2, 3, 4-Trinor-1, 5-inter-m-phenylen-16-amino-$PGI_2$-Natriumsalz, 2, 3, 4-Trinor-1, 5-inter-m-phenylen-16-acetamido-$PGI_2$-Natriumsalz, 2, 3, 4-Trinor-1, 5-inter-m-phenylen-16-benzoylamino-$PGI_2$-Natriumsalz, 2, 3, 4-Trinor-1, 5-inter-m-phenylen-16-tosylamino-$PGI_2$-Natriumsalz, 2, 3, 4, 17, 18, 19, 20-Heptanor-1, 5-inter-m-phenylen-16-amino-$PGI_2$-Natrium-salz, 2, 3, 4, 18, 19, 20-Hexanor-1, 5-inter-m-phenylen-16-amino-17-phenyl-$PGI_2$-Natriumsalz, 5(Z)-2, 3, 4, 17, 18, 19, 20-Heptanor-1, 5-inter-m-phenylen-16-phenoxy-$PGI_2$-Dicyclohexylaminsalz, 5(Z)-2, 3, 4, 17, 18, 19, 20-Heptanor-1, 5-inter-m-phenylen-16-phenoxy-$PGI_2$-Ammoniumsalz, 5(Z)-2, 3, 4, 17, 18, 19, 20-Heptanor-1, 5-inter-m-phenylen-16-phenoxy-$PGI_2$-Dimethylaminsalz, 5(Z)-2, 3, 4, 17, 18, 19, 20-Heptanor-1, 5-inter-m-phenylen-16-phenoxy-$PGI_2$-Tetrabutylammoniumsalz, 5(Z)-2, 3, 4, 17, 18, 19, 20-Heptanor-1, 5-inter-m-phenylen-16-phenoxy-$PGI_2$-tris-(hydroxymethyl)-Methylammoniumsalz.

Die Erfindung betrifft auch die Herstellung der 2,3, 4-Trinor-1,5-in- ter-m-phenylen-$PGI_2$-Derivate der allgemeinen Formel I

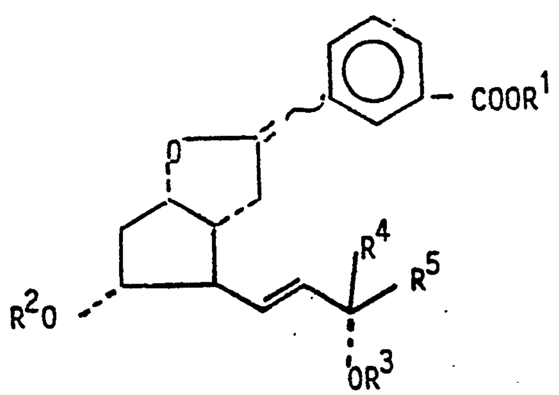

(I) ,

worin

$R^1$, $R^2$, $R^3$, $R^4$, $R^5$, Z und $R^6$ die oben angegebene Bedeutung haben, die dadurch gekennzeichnet ist, daß man bicyclische Lactole der allgemeinen Formel II

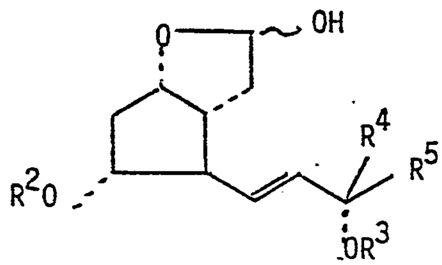

(II),

worin

$R^4$, $R^5$, Z und $R^6$ die oben angegebene Bedeutung haben und $R^2$ und $R^3$ unabhängig voneinander Wasserstoff, $C_{1-4}$-Alkanoyl, gegebenenfalls substituiertes Benzoyl, Äthoxyäthyl, Tetrahydropyranyl oder Tri-$C_{1-4}$-alkylsilyl bedeuten, mit einem Phosphoniumsalz der allgemeinen Formel III

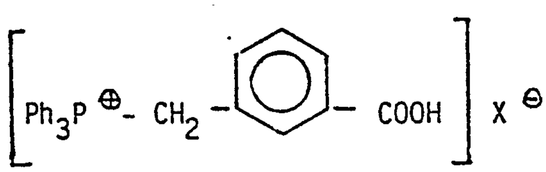

(III)

worin

Ph Phenyl und

X ein einwertiges Anion bedeute,

und einer starken Base - wobei sich das entsprechende Phosphoran bildet - in einem dipolaren aprotischen Lösungsmittel umsetzt, das erhaltene Prostaglandin-Derivat der allgemeinen Formel IV

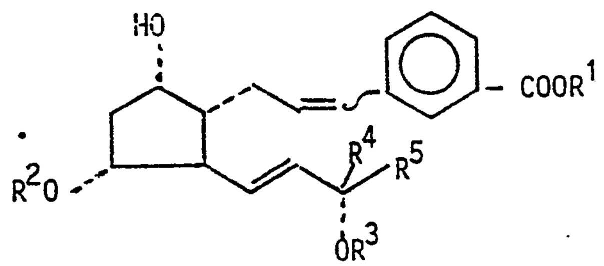

(IV),

worin

$R^2$, $R^3$, $R^4$, $R^5$, Z und $R^6$ die oben angegebene Bedeutung haben und $R^1$ Wasserstoff ist,

gewünschtenfalls nach Überführung in einen Ester, in dem $R^1$ $C_{1-4}$-Alkyl ist, und - sofern der Substituent $R^5$ eine freie Aminogruppe enthält - nach Acylierung mit einem eine gegebenenfalls halogensubstituierte $C_{1-4}$-Alkanoyl-, Benzoyl- oder Tosylgruppe enthaltenden Acylierungsmittel,

durch Behandeln mit einem Halogenierungsmittel der allgemeinen Formel

E - X,

worin

X Chlor, Brom oder Jod und

E Chlor, Brom, Jod, Acetyl, Trifluoracetyl oder den N-Succinimidrest bedeuten,

in einem mit Wasser nicht mischbaren organischen Lösungsmittel, das auch in einem mit Wasser gebildeten Zweiphasensystem vorliegen kann, in An- oder Abwesenheit eines Säureacceptors cyclisiert und aus dem erhaltenen 5-Halogen-$PGI_1$-Derivat der allgemeinen Formel V

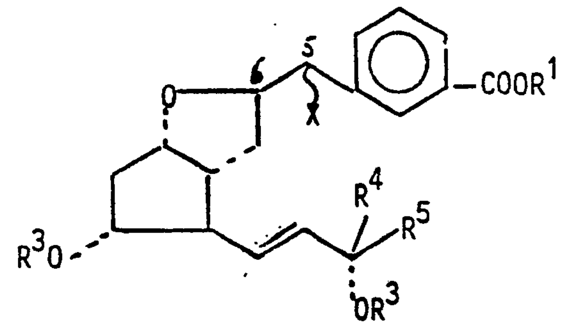

(V) ,

worin
$R^1$, $R^2$, $R^3$, $R^4$, $R^5$, $R^6$ und X die oben angegebene Bedeutung haben
und
Z eine von der freien Aminogruppe verschiedene Bedeutung hat. Durch Behandeln mit basischen Reagentien in 5, 6-Stellung Halogenwasserstoff abspaltet, wobei das erhaltene $PGI_2$-Derivat der allgemeinen Formel I gewünschtenfalls durch Verseifung, Salzbildung, Acylieren oder Desacylieren in ein anderes unter die allgemeine Formel I fallendes PGI-Derivat übergeführt werden kann.

Bei den gemäß Erfindung als Ausgangsmaterialien verwendeten bicyclischen Lactolen der allgemeinen Formel II handelt es sich um die entsprechend substituierten Derivate des 3, 3aβ-4, 5, 6aβ-Hexahydro-2-hydroxy-4β-(3-hydroxy-1-trans-octenyl)-5α-hydroxy-2H-cyclopenta[b]-furans, eines bekannten Prostaglandin-Zwischenproduktes. Über die Herstellung dieser Zwischenprodukte sind mehrere zusammenfassende Arbeiten erschienen (z.B. Prostaglandin Research, 237-239, Editor P. Crabbe, Academic Press, 1977; J.S. Bindra: Prostaglandin Synthesis, Academic Press 1977, 462 uff., ferner in Kapitel 10).

Die Umsetzung zwischen dem bicyclischen Lactol der allgemeinen Formel II und dem Triphnyl-m-carboxybenzyl-phosphonium-halogenid der allgemeinen Formel III in Gegenwart einer starken Base - wie Natriumhydrid, Natriumamid, Natriumethylat - unter Bildung des entsprechenden 2, 3, 4-Trinor-1, 5-inter-m-phnylen-$PGF_{2\alpha}$-Derivats der allgemeinen Formel IV erfolgt nach der Wittig-Reaktion. Hierbei bildet sich unter Einwirkung der starken Base aus dem Phosphoniumsalz der allgemeinen Formel III in situ das entsprechende Phosphoran, das dann mit dem bicyclischen Lactol der allgemeinen Formel II in Reaktion tritt. Die Umsetzung wird in dipolaren, aprotischen Lösungsmitteln, wie Dimethylsulfoxyd, N,N-Dimethylformamid, Hexamethylphosphorsäuretriamid, durchgeführt.

Weist das so erhaltene $PGF_{2\alpha}$-Derivat der allgemeinen Formel IV am Substituenten $R^5$ eine freie Aminogruppe auf ($Z = NH_2$), so kann diese vor der Weiterverarbeitung durch Behandeln mit einem Acylierungsmittel, das eine gegebenenfalls durch Halogen substituierte $C_{1\text{-}4}$-Alkanoyl-, Alkoxycarbonyl-, Benzoyl-, Tosyl-, Benzyloxycarbonyl- oder p-Nitrophenoxycarbonylgruppe enthält, acyliert werden. Vorzugsweise werden als Acylierungsmittel Acetylchlorid, Tosylchlorid, Benzoylchlorid oder Trifluoressigsäure verwendet.

Die Cyclisierung des bei der Wittig-Reaktion erhaltenen $PGF_{2\alpha}$-Deri-vats der allgemeinen Formel IV zum 5-Halogen-$PGI_1$-Derivat der allgemeinen Formel V wird durch Behandlung mit einem Halogenierungsmittel der allgemeinen Formel E - X durchgeführt. Einige Beispiele für geeignete Halogenierungsmittel E - X sind: Jod, Brom, N-Bromsuccinimid, N-Jodsuccinimid, N-Chlorsuccinimid, wobei dem elementaren Jod und N-Bromsuccinimid der Vorzug gegeben wird. Ob die Cyclisierungsreaktion in An- oder Abwesenheit eines Säureacceptors durchgeführt wird, hängt von der Wahl des Halogenierungsmittels ab. Während es zweckmäßig ist, bei Verwendung von Halogenierungsmitteln, wie dem elementaren Jod, die Cyclisierung in Gegenwart eines Säureacceptors, wie Natriumhydrogencarbonat, durchzuführen, kann die Cyclisierung mit den N-Halogensuccinimiden ohne Säureacceptor durchgeführt werden.

Die Überführung der 5-Halogen-$PGI_1$-Derivate der allgemeinen Formel V durch Halogenwasserstoffabspaltung und unter Ausbildung der 5, 6-Dop-pelbindung in die $PGI_2$-Derivate der allgemeinen Formel 1 erfolgt durch Behandlung mit basischen Reagentien. Einige Beispiele hierfür sind: Alkalicarbonate (Kaliumcarbonat), Alkalihydrogencarbonate (Natriumhydrogencarbonat), Alkalihydroxyde (Kaliumhydroxyd), aber auch tertiäre Amine, wie Triäthylamin, Pyridin oder aber 1, 5-Diazabicyclo[4, 3, 0]non-5-en (DBN) oder 1, 5-Diazabicyclo[5, 4, 0]undec-5-en (DBU).

Die erhaltenen Produkte werden durch Säulenchromatographie an Silicagel gereinigt.

Zur Herstellung von Verbindungen, in denen $R^2$ und $R^3$ nicht Wasserstoff sind, kann man auch so vorgehen, in den 5-Halogen $PGI_1$-Derivaten der allgemeinen Formel V vor der Halogenwasserstoffabspaltung die Hydroxylgruppen in 11- und 15-Stellung entsprechend schützt. Man kann aber auch die Acylierung, Benzoylierung, Äthoxyäthylierung etc. der Hydroxylgruppen in 11- und 15-Stellung in dem $PGI_2$-Derivat der allgemeinen Formel I durchführen.

Soll $R^1$ in den Verbindungen der Erfindung $C_{1\text{-}4}$-Alkyl sein, werden die entsprechenden Carbonsäuren

zweckmäßig mit den entsprechenden Diazoalkanen umgesetzt. Soll $R^1$ Alkali oder primäres, sekundäres, tertiäres oder quaternäres Ammonium sein, werden zweckmäßig die entsprechenden Säuren mit den entsprechenden Basen oder Aminen umgesetzt. Verbindungen in denen $R^1$ Alkali ist, können auch durch Hydrolyse der Ester erhalten werden. Zur Herstellung von Verbindungen, in denen Z eine freie Aminogruppe bedeutet, wird die vorher eingeführte Schutzgruppe in der letzten Verfahrensstufe aus der 16-Aminogruppe abgespalten. Einige Beispiele für geeignete Schutzgruppen sind: gegebenenfalls durch Halogen substituierte $C_{1-4}$-Alkanoylgruppen, Alkoxycarbonyl-, Benzoyl-, Tosyl-, Benzyloxycarbonyl- und p-Nitrophenoxycarbonylgruppen, die sich in an sich bekannter Weise leicht abspalten lassen.

Aus Verbindungen der allgemeinen Formel II, in denen Z eine Trifluoracetylaminogruppe bedeutet, wird während der Halogenwasserstoffabspaltung durch Basen die Trifluoracetylgruppe abgespalten, wobei Verbindungen der allgemeinen Formel I mit einer freien Aminogruppe erhalten werden.

Unter einer $C_{1-4}$-Alkylgruppe ist in der vorliegenden Beschreibung die Methyl-, Äthyl-, n- und Isopropyl-, n-, Iso-, sec.- und tert.-Butylgruppe zu verstehen. Die gegebenenfalls durch Halogen sunstituierten $C_{1-4}$-Alkanoylgruppen stellen von den entsprechenden Alkanen ableitbare Alkanoylgruppen dar, die einfach oder mehrfach halogensubstituiert sein können. Beispiele für die Substituenten sind: Fluor, Chlor, Brom und Jod. Die Benzoylgruppe kann durch Halogen oder Phenyl substituiert sein. In den Trialkylsilylgruppen können die Alkylgruppen gleich oder verschieden sein. Beispiele für die $C_{4-6}$-Alkylgruppen sind die geraden und verzweigten Isomere der Butyl-, Pentyl-, Hexylgruppe.

Das Alkaliion $R^1$ kann ein Natrium-, Kalium- oder Lithiumion sein. Unter prim., sec., tert. und quat. Ammoniumionen sind die linearen oder cyclischen, ein oder mehrere Stickstoffatome enthaltenden organischen Amine zu verstehen, in denen das Stickstoffatom durch eine, zwei, drei oder vier $C_{1-4}$-Alkylgruppen oder durch eine $C_{5-8}$-Cycloalkylgruppe, z.B. die Cyclopentyl-, Cyclohexyl-, Cycloheptyl- oder Cyclooctylgruppe, substituiert ist. Die am Stickstoffatom gebundenen Alkylgruppen können ebenfalls substituiert sein, vorzugsweise durch Hydroxylgruppen.

Die Erfindung wird durch die folgenden Beispiele näher erläutert.

**Beispiel 1**

2, 3, 4, 17, 18, 19, 20-Heptanor-1, 5-inter-m-phenylen-11, 15-bis-tetrahydro-pyranyloxy-16-phenoxy-$PGF_{2\alpha}$-Methylester

(Formel IV: $R^1$ = $CH_3$, $R^2$, $R^3$ = Tetrahydropyranyl, $R^4$ = H, $R^5$ = $-CH_2-O-Ph$) 250 mg (10,41 mM) Natriumhydrid werden in einen gut getrockneten, mit Magnetrührer, Thermometer und einer Gummikappe ausgerüsteten Dreihalskolben eingewogen. Der Kolben wird mit Stickstoff ausgespült, dann mit 5 ml trockenem Dimethylsulfoxyd beschickt und mit einer Stickstoffgasflasche verbunden. Es wird ein langsamer Stickstoffstrom durch den Kolben geleitet, wobei durch einen Quecksilberverschluß in der Leitung im Kolben ein schwacher $N_2$-Überdruck aufrechterhalten wird. Die Temperatur der Suspension wird langsam auf 60°C erhöht, wobei eine Gasentwicklung zu beobachten ist. Nach deren Aufhören (etwa 30 min) wird das Gemisch auf 70°C erhitzt und bei dieser Temperatur weitere 30 Minuten gerührt. Man erhält eine dunkelgraue Lösung, der man bei 15°C 2,45 g (5,68 mM) im Vakuum getrocknetes m-Carboxybenzyl-triphenyl-phosphoniumchlorid zufügt. Die erhaltene rote Lösung wird bei Raumtemperatur eine halbe Stunde gerührt, sodann mit der Lösung von 1,57 g (2,59 mM) 3, 3aß, 4, 5, 6, 6aß-Hexahydro-2-hydroxy-4ß-(3-tetrahydropyranyloxy-4-phenoxy-butenyl)-5α-tetrahydropyranyloxy-2H-cyclopenta[b]furan in 500 ml wasserfreiem Dimethylsulfoxyd versetzt, bei 60°C eine Stunde gerührt und dann in ein Gemisch aus 20 g Eis und 50 ml Wasser gegossen. Die Lösung extrahiert man 2 x mit 10 ml Äther, stellt den pH-Wert der wäßrigen Phase mit 2n wäßriger Natriumhydrogensulfatlösung auf 4 - 5 ein, extrahiert die wäßrige Phase 4 x mit 20 ml Äther und dann 2 x mit 20 ml Äthylacetat, trocknet die vereinigten organischen Phasen über Natriumsulfat, filtriert sie und dampft das Lösungsmittel unter vermindertem Druck ab. Das erhaltene Rohprodukt (2,5 g) wird in 20 ml Äther gelöst und bei 0°C tropfenweise unter Rühren so lange mit Diazomethanlösung versetzt, bis die gelbe Farbe des zugetropften Diazomethans nicht mehr verschwindet. Der gebildete Methylester wird schichtchromatographisch überprüft. In einem Gemisch von Benzol, Dioxan und Essigsäure (Verhältnis 20: 10: 1)auf einer Silicagel G Schicht beträgt der $R_f$-Wert der bei der Wittig-Reaktion entstehenden Säure 0,52, der des aus dieser bereiteten Methylesters 0,73. (Falls die Bildung des Methylesters nicht quantitativ war, wird weitere ätherische Diazomethanlösung zugesetzt.) Der rohe Methylester wird nach dem Abdestillieren des Äthers auf einer Silicagelsäule mit einem Benzol-Dioxan-Essigsäuregemisch (20: 10: 1) chromatographiert, die auf dem Schichtdiagramm dem Fleck bei $R_f$ 0,73 entsprechenden Fraktionen werden gesammelt und eingedampft, setzt.) Der rohe Methylester wird nach dem Abdestillieren des Äthers auf einer Silicagelsäule mit einem Benzol-Dioxan-Essigsäuregemisch (20: 10: 1) chromatographiert, die auf dem Schichtdiagramm dem Fleck bei $R_f$ 0,73 entsprechenden Fraktionen werden gesammelt und eingedampft, wobei man 1,426 g (70 %) der Titelverbindungen erhält. $R_f$ in dem oben angegebenen System: 0,73.

**Beispiel 2**

2, 3, 4, 17, 18, 19, 20-Heptanor-1, 5-inter-m-phenylen-16-phenoxy-PGF-$2_{2\alpha}$ Methylester
(Formel IV: $R^1$ = $CH_3$, $R^2$, $R^3$, $R^4$ = H, $R^5$ = -$CH_2$-O-Ph)
Es wird wie in Beispiel 1 verfahren, jedoch mit dem Unterschied, daß man dem Reaktionsgemisch die Lösung von 350 mg (1,29 mM) 3, 3aß, 4, 5, 6, 6aß-Hexahydro-2-hydroxy-4ß-(3-hydroxy-4-phenoxy-1-trans-butenyl)-5α-hydroxy-2H-cyclopenta[b]furan in 450 ml trockenem Dimethylsulibxyd zusetzt. Es werden 401,9 mg (77,5 %) der im Titel genannten Verbindung erhalten.
$^1$H-NMR($CDCl_3$,δ): 6,85 - 7,5 (m, 7H, aromatisch), 7,5 - 8,05 (m, 2H, aromatisch), 5,25 - 6,75 (m, 4H, Olefinprotonen), 3,92 (s, 3H, $OCH_3$).
In analoger Weise werden hergestellt: 2, 3, 4, 17, 18, 19, 20-Heptanor-1, 5-inter-m-phenylen-16-(m-trifuormethylphenoxy)-$PGF_{2\alpha}$-Methylester, $^1$H-NMR($CDCl_3$,δ): 7,1 - 7,5 (m, 5H, aromatische Protonen), 7,5 - 8,05 (m, 3H, aromatische Protonen),
2, 3, 4-Trinor-1, 5-inter-m-phenylen-20-methyl-$PGF_{2\alpha}$-Methylester $^1$H-NMR($CDCl_3$,δ): 0,89 (t, 3H, $CH_3$), 3,90 (s, 3H, -$OCH_3$), 5,23 - 6,7
(m, 4H, Olefinprotonen), 7,2 - 7,55 (m, 2H, aromatische Protonen), 7,6 - 8,05 (m, 2H, aromatische Protonen),
$R_f$ = 0,34 (Benzol, Dioxan, Essigsäure = 20: 10: 1),
2, 3, 4-Trinor-1, 5-inter-phenylen-20-äthyl-$PGF_{2\alpha}$-Methylester
$R_f$ = 0,35 (Benzol/ Dioxan/ Essigsäure = 20: 10: 1).

**Beispiel 3**

2, 3, 4, 18, 19, 20-Hexanor-1, 5-inter-m-phenylen-11, 15-bis-tetrahydro-pyranyloxy-16-acetylamino-17-phenyl-$PGF_{2\alpha}$-Methylester

(Formel IV: $R^1$ = $CH_3$, $R^2$, $R^3$ = Tetrahydropyranyl, $R^4$ = H, $R^5$ = $\underset{Z}{-CH}-R^6$
($R^6$ =-$CH_2$-Ph, Z = -NH-Ac))

Es wird wiein Beispiel 1 verfahren, jedoch mit dem Unterschied, daß man dem Reaktionsgemisch die Lösung von 1,37 g (2,59 mM) 3,3aß,4,5,6, 6aß-Hexahydro-2-hydroxy-4ß-(3-tetrahydropyranyloxy-4-acetamido-5-phe-nyl-1-trans-pentenyl)-5α-tetrahydropyranyloxy-2H-cyclopenta[b]-furan in 500 µ1 trockenem Dimethylsulfid zusetzt. Man erhält 1,426 g (70 %) Produkt. In einem Benzol-Dioxan-Essigsäure-Gemisch (20: 10: 1) an einer analytischen Silicagelschicht (Merck G) beträgt der $R_f$-Wert 0,05.

**Beispiel 4**

2, 3, 4-Trinor-1, 5-inter-m-phenylen-16-amino-$PGF_{2\alpha}$-Methylester

(Formel IV: $R^1$ = $CH_3$, $R^2$, $R^3$, $R^4$ = H, $R^5$ = $\underset{Z}{-CH}-R^6$ ($R^6$ = -$C_4H_9$, Z = -$NH_2$))

a) Es wird wie in Beispiel 2 verfahren, jedoch mit dem Unterschied, daß man dem Reaktionsgemisch die Lösung von 0,497 g (1,29 mM) 3, 3aβ, 4, 5, 6, 6aβ-Hexahydro-2-hydroxy-4ß-[3-hydroxy-4-(tert.-butoxycarbonyl-amino)-1-trans-octenyl]-5α-hydroxy-2H-cyclopenta[b]furan in 400 µl trockenem Dimethylsulfoxyd zusetzt. Man erhält 0,450 g (87 %) 2, 3, 4-Trinor-1,5-inter-m-phenylen-16-(tert:butoxycarbonylamino)-$PGF_{2\alpha}$ -Methylester. $R_f$ (Silicagel G: Äthylacetat / Benzol = 4: 1) = 0,31.
In der gleichen Weise werden hergestellt: 2, 3, 4, 17, 18, 19, 20-Heptanor-1,5-inter-m-phenylen-16-phenyl-16-(tert.-butoxycarbonylamino)-$PGF_{2}\alpha$-Methylester. $R_f$ = 0,35, (Äthylacetat/ Benzol = 4: 1), und 2,3,4,18,19,20-Hexanor-1,5-inter-m-phenylen-16-(tert.-butoxycarbonyl-amino)-17-phenyl-$PGF_{2\alpha}$-Methylester, $R_f$ = 0,36 (Äthylacetat / Benzol = 4: 1).
b) 0,450 g (0,87 mM) 2, 3, 4-Trinor-1, 5-inter-m-phenylen-16-(tert.-but-oxycarbonylamino)-$PGF_{2\alpha}$-Methylester löst man in 5 ml Essigsäure und leitet in die Lösung 1,05 Äquivalente trockenes Salzsäuregas ein. Das Ende der Reaktion wird durch Gasentwicklung (Isobutylen) angezeigt. Danach wird das Reaktionsgemisch mit 50 ml

Äthylacetat verdünnt, der pH-Wert unter intensivem Rühren mit ln Natronlauge auf 8 - 9 eingestellt, die wäßrige Phase abgetrennt und die organische Phase nach Trocknen über wasserfreiem Natriumsulfat zur Trockene eingedampft. Das Rohprodukt wird an einer Silicagelsäule mit einem Gemisch aus Isopropanol und Wasser (Verhältnis 8: l)chromatographiert. Die Fraktionen mit $R_f$ = 0,4 (Isopropanol / Wasser = 7: 2) werden gesammelt und eingedampft. Es werden 0,291 g der Titelverbindung erhalten. In der gleichen Weise werden hergestellt: 2, 3, 4, 17, 18, 19, 20-Heptanor-1,5-inter-m-phenylen-16-amino-16-phenylen $PGF_{2\alpha}$-Methylester ($R_f$ = 0,45; Isopropanol Wasser = 7: 2) und-2, 3, 4, 18, 19, 20-Hexanor-1, 5-inter-m-phenylen-16-amino-17-phenyl-$PGF_{2\alpha}$-Methylester ($R_f$ = 0,47; Isopronanol / Wasser = 7: 2).

**Beispiel 5**

2, 3, 4-Trinor-1, 5-inter-m-phenylen-16-acetylamino-$PGF_{2\alpha}$ -Methylester

(Formel IV: $R^1 = CH_3$, $R^2$, $R^3$, $R^4$ = H, $R^5$ = $-\underset{Z}{CH}-R^6$ ($R^6 = -C_4H_9$, Z = -NH-Ac))

2,5 g (5,92 mM) 16-Amino-2, 3, 4-trinor-1, 5-inter-m-phenylen-$PGF_{2\alpha}$-Methylester löst man in 3 ml wasserfreiem Pyridin, kühlt die Lösung auf 0°C und tropft 0,98 g (0,79 ml; 12,5 mM) Acetylchlorid zu. Das Reaktionsgemisch wird bei 0°C 30 Minuten gerührt und dann mit 50 ml Äthylacetat verdünnt. Die organische Phase wird mit 10 ml 1n Salzsäure gewaschen, über wasserfreiem Magnesiumsulfat getrocknet und nach dem Filtrieren das Lösungsmittel unter vermindertem Druck abgedampft. Es werden 2,36 g (86 %) 2, 3, 4-Trinor-1, 5-inter-m-phenylen-16-acetylamino-$P6F_{2\alpha}$-Methylester erhalten. $R_f$ = 0,7 (Isopropanol / Wasser = 7: 2).

In der gleichen Weise werden hergestellt: 2, 3, 4-Trinor-1, 5-inter-m-phenylen-16-benzoylamino-$PGF_{2\alpha}$ -Methylester
($R_f$ = 0,67; Isopropanol / Wasser = 7: 2) und
2, 3, 4-Trinor-1,5-inter-m-phenylen-16-tosylamino-$PGF_{2\alpha}$-Methylester ($R_f$ = 0,61; Isopropanol / Wasser = 7: 2).

**Beispiel 6**

2, 3, 4, 17, 18, 19, 20-Heptanor-1, 5-inter-m-phenylen-5-jod-16-phenoxy-$PGI_1$-Methylester
(Formel V: $R^1 = CH_3$, $R^2$, $R^3$, $R^4$ = H, $R^5 = -CH_2-O-Ph$, X = J)

355 mg (0,81 mM) 2, 3, 4, 17, 18, 19, 20-Heptanor-1, 5-inter-m-phenylen-16-phenoxy-$PGF_{2\alpha}$-Methylester löst man in 5 ml wasserfreiem Dichlormethan und gibt zu der Lösung 4,05 ml IM Natriumhydrogencarbonatlösung. Das zweiphasige Gemisch wird bei 0°C intensiv gerührt und bei dieser Temperatur innerhalb von 15 Minuten mit 16,4 ml (1,62 mM) einer 0,0976 M Jodlösung in Dichlormethan versetzt. Nach Beendigung des Zusatzes wird das Gemisch bei Raumtemperatur 3 Stunden gerührt und dann bis zur Entfärbung mit 5 %iger wäßriger Natriumthiosulfatlösung versetzt. Die wäßrige Phase wird 3 x mit 50 ml Äther und dann mit 20 ml Äthylacetat extrahiert. Die vereinigten organischen Phasen trocknet man über Natriumsulfat, filtriert sie und destilliert dann das Lösungsmittel unter vermindertem Druck ab, wobei man 530 mg Rohprodukt erhält.

Das Produkt wird an einer mit 100 g Silicagel (Merck Art. No. 7734, Teilchengröße 0,03 - 0,2 mm) gefüllten Säule mit Äthylacetat chromatographiert, wobei die Fraktionen auf der analytischen Dünnschicht (Merck Art. No. 5715) mit den $R_f$-Werten 0,49 und 0,47 vereinigt werden. Das Lösungsmittel wird unter vermindertem Druck abdestilliert. Die beiden Produktfraktionen haben zusammen ein Gewicht von 493 mg (87 %). Der dünnschichtchromatographisch (Silicagel G.Merck Art. No. 5715) mit Äthylacetat gemessene $R_f$-Wert des Endo- bzw. Exoisomeren beträgt 0,49 bzw. 0,47.

In der gleichen Weise werden hergestellt: 2, 3, 4, 17, 18, 19, 20-Heptanor-1, 5-inter-m-phenylen-5-jod-16-(3-trifluor-methyl-phenoxy)-$PGI_1$-Methylester ($R_f$ = 0,51 und 0,48, Äthylacetat), 2, 3, 4-Trinor-1, 5-inter-m-phenylen-5-jod-16-acetylamino-$PGI_1$-Methylester ($R_f$ = 0,6, Isopropanol / Wasser = 7: 2), 2, 3, 4-Trinor-1, 5-inter-m-phenylen-5-jod-16-benzoylamino-$PGI_1$-Methyl-ester ($R_f$ =0,63, Isopropanol / Wasser = 7: 2), 2, 3, 4-Trinor-1, 5-inter-m-phenylen-5-jod-16-tosylamino-$PGI_1$-Methylester ($R_f$ = 0,56, Isopropanol / Wasser = 7: 2),
2, 3, 4-Trinor-1, 5-inter-m-phenylen-5-jod-20-methyl-$PGI_1$-Methylester ($R_f$ = 0,43 und 0,40, Äthylacetat), 2, 3, 4-Trinor-1, 5-inter-m-phenylen-5-jod-20-äthyl-$PGI_1$-Methylester ($R_f$ = 0,43 und 0,41, Äthylacetat), 2, 3, 4, 17, 18, 19, 20-Heptanor-1, 5-inter-m-phenylen-5-jod-11, 15-diacetoxy = 16-phenoxy-$PGI_1$-Methylester ($R_f$ = 0,7 und 0,72, Benzol / Äthylacetat = 1: 1).

**Beispiel 7**

2, 3, 4-Trinor-1, 5-inter-m-phenylen-5-brom-16-amino-$PGI_1$-Methylester

(Formel V: $R^1 = CH_3$, $R^2$, $R^3$, $R^4$ = H, $R^5$ = CH-$R^6$ ($R^6 = C_4H_9$, Z = $NH_2$))

460 mg (1,1 mM) 2, 3, 4-Trinor-1,5-inter-m-phenylen-16-amino-$PGF_{2\alpha}$-Methylester löst man in 3 ml wasserfreiem Triäthylamin, kühlt die Lösung auf 0°C, gibt dann 160 $\mu$l (1,15 mM) wasserfreie Trifluoressigsäure zu, rührt das Reaktionsgemisch bei 0°C eine halbe Stunde und verdünnt es dann mit 50 ml Äthylacetat. Die organische Phase wird 2 x mit 10 ml 1n Oxalsäure gewaschen, über wasserfreiem Magnesiumsulfat getrocknet und nach dem Filtrieren von dem Lösungsmittel durch Destillation unter vermindertem Druck befreit.

Der erhaltene rohe 2, 3, 4-Trinor-1, 5-inter-m-phenylen-16-(trifluor-acetamido)-$PGF_{2>\alpha}$-Methylester wird in 5 ml eines wasserfreien Gemisches von Chloroform und Tetrahydrofuran (1: 1) gelöst, die erhaltene Lösung auf -78°C gekühlt (Kältemischung aus Trockeneis und Aceton) und unter Schutzgasatmosphäre gerührt. Nach Zusatz von 215,4 mg (1,21 mM) festem N-Bromsuccinimid wird das Reaktionsgemisch bei -78°C nach 10 Minuten gerührt, dann wird die Kühlung abgesetzt. Man läßt die Temperatur des Reaktionsgemisches auf Raumtemperatur ansteigen und rührt bei dieser Temperatur 30 Minuten. Dann wird das Reaktionsgemisch mit 50 ml Chloroform verdünnt und 3 x mit 20 ml Wasser gewaschen. Die organische Phase wird über Natriumsulfat getrocknet und nach dem Filtrieren von dem Lösungsmittel durch Destillation unter vermindertem Druck befreit. Als Rückstand werden 680 mg 2, 3, 4-Trinor-1, 5-inter-m-phenylen-5-brom-16-(trifluoracetamido)-$PGI_1$-Methylester erhalten ($R_f$ = 0,35; Äthylacetat / Benzol = 3: 1).

Das Rohprodukt wird mit 2 ml einer 0,03 molaren Kaliumhydrogencarbonatlösung - Lösungsmittel ein 95: 5 - Gemisch aus Wasser und Methanol - eine Stunde gerührt und dann die wäßrige Phase 5 x mit 5 ml Äthylacetat extrahiert. Die vereinigten organischen Phasen werden über Kaliumcarbonat getrocknet, filtriert und dann unter vermindertem Druck eingedampft. Es werden 395 mg (72 %) der Titelverbindung erhalten ($R_f$ = 0,45; Isopropanol / Wasser = 7: 2).

In der gleichen Weise werden hergestellt: 2, 3, 4, 17, 18, 19, 20-Heptanor-1, 5-inter-m-phenylen-5-brom-16-amino-16-phenyl-$PGI_1$-Methylester ($R_f$ = 0,49; Isopropanol / Wasser;7: 2) und 2, 3, 4, 18, 19, 20-Hexanor-1, 5-inter-m-phenylen-5-brom-16-amino-17-phenyl-$PGI_1$-Methylester ($R_f$ = 0,47; Isopropanol / Wasser = 7: 2).

**Beispiel 8**

2, 3, 4, 17, 18, 19, 20-Heptanor-1, 5-inter-m-phenylen-16-phenoxy-$PGI_2$-Methyl-ester
(Formel I: $R^1 = CH_3$, $R^2$, $R^3$, $R^4$ = H, $R^5$ = -$CH_2$-O-Ph)

Zu 230,4 mg (0,40 mM) 2, 3, 4, 17, 18, 19, 20-Heptanor-1, 5-inter-m-phenylen-5-jod-16-phenoxy-$PGI_1$-Methylester gibt man 2 ml destilliertes 1, 5-Di-azabicyclo[4, 3, 0]non-5-en, rührt das Gemisch bei 50°C eine Stunde, verdünnt dann mit 50 ml Äther und wäscht die organische Phase 2 x mit 20 ml Wasser. Anschließend wird über Natriumsulfat getrocknet, filtriert und der Äther unter vermindertem Druck abdestilliert. Es werden 210 mg Rohprodukt erhalten.

Das Rohprodukt wird an einer mit 60 g Silicagel (Merck Art.No. 7734, Korngröße 0,03 - 0,2 mm) gefüllten Säule mit einem Gemisch von Äther und Aceton (Verhältnis 3: 1) chromatographiert. Die Fraktionen, die den dünnschichtchromatographisch (Merck Art. No. 5715, Fließmittel: Äthylacetat) ermittelten $R_f$-Werten 0,49 und 0,51 entsprechen, werden gesammelt und durch Destillation vom Lösungsmittel befreit. Insgesamt werden 152,1 mg (85,4 %) der Titelverbindung erhalten. Dünnschichtchromatographie: das Z-Isomer hat einen $R_f$-Wert von 0,51, das E-Isomer von 0,49 (Silicagel G.Merck Art. No. 5717).

In der gleichen Weise werden folgende Verbindungen hergestellt: 2, 3, 4, 17, 18, 19, 20-Heptanor-1, 5-inter-m-phenylen-16-(3-trifluormethyl-phenoxy)-$PGI_2$-Methylester ($R_f$ = 0,51 und 0,53; Äthylacetat), 2, 3, 4-Trinor-1,5-inter-m-phenylen-16-amino-$PGI_2$-Methylester ($R_f$ = 0,45; Isopropanol / Wasser = 7: 3),

2, 3, 4-Trinor-1, 5-inter-m-phenylen-16-acetamido-$PGI_2$-Methylester ($R_f$-0,70, Isopropanol / Wasser = 8: 3),

2, 3, 4-Trinor-1, 5-inter-m-phenylen-16-benzoylamino-$PGI_2$-Methylester ($R_f$ = 0,74, Isopropanol / Wasser = 7: 3),

2, 3, 4, 17, 18, 19, 20-Heptanor-1, 5-inter-m-phenylen-16-amino-16-phenyl-$PGI_2$-Methylester ($R_f$ = 0,49, Isopropanol / Wasser = 7: 3),

2, 3, 4, 18, 19, 20-Hexanor-1,5-inter-m-phenylen-16-amino-17-phenyl-$PGI_2$-Methylester ($R_f$ = 0,53, Isopropanol / Wasser = 7: 3).

**Beispiel 9**

5(Z)-2, 3, 4, 17, 18, 19, 20-Heptanor-1,-5-inter-m-phenylen-16-phenoxy:$PGI_2$-Natriumsalz
(Formel I: $R^1$ = Na, $R^2$, $R^3$, $R^4$ = H, $R^5$ = $-CH_2-O-Ph$)

338,7 mg (0,78 mM) 5(Z)-2, 3, 4, 17, 18, 19, 20-Heptanor-1, 5-inter-m-phenylen-16-phenoxy-$PGI_2$-Methylester löst man in 2 ml Methanol, gibt zu der Lösung bei Raumtemperatur 1,86 ml (0,93 mM) 0,5 molare methanolische Natronlauge, rührt das Gemisch bei Raumtemperatur 6 Stunden und destilliert dann das Methanol unter vermindertem Druck ab. Der feste Rückstand wird in Petroläther suspendiert und dann dekantiert. Es werden 340,4 mg (98 %) der Titelverbindung erhalten. $R_f$ = 0,43 (Benzol / Dioxan / Essigsäure = 20: 10: 1) $^1H$-NMR(Deuteromethanol δ: 5,32 (breites s, 1H, Protonen in 5-Stellung),

5,45 - 5,77 (m, 2H, Olefinprotonen), 6,9 - 7,5 (m, 6H, aromatische Protonen), 7,5 - 8,15 (m, 3H, aromatische Protonen).

In der gleichen Weise werden die folgenden Verbindungen hergestellt: 5(E)-2, 3, 4, 17, 18, 19, 20-Heptanor-1, 5-inter-m-phenylen-16-phenoxy-$PGI_2$-Natriumsalz ($R_f$ = 0,41, Benzol / Dioxan / Essigsäure = 20: 10: 1), $^1H$-NMR (Deuteromethanol)δ =

5,5 - 5,7 (m, 2H, Olefinprotonen), 5,95 (breites s, 1H, Protonen in 5-Stellung), 6,9 - 8,15 (m, 9H, aromatische Protonen), 5(E, Z)-2, 3, 4, 17, 18, 19, 20-Heptanor-1, 5-inter-m-phenylen-16-(3-trifluor-methyl-phenoxy)-$PGI_2$-Natriumsalz ($R_f$ = 0,47, Benzol/ Dioxan / Essigsäure = 20: 10: 1), 5(Z)-2, 3, 4-Trinor-1, 5-inter-m-phenylen-20-methyl-$PGI_2$-Natriumsalz $^1H$-NMR(Deuteromethanol)δ = 0,89 (t, 3H, $CH_3$-) 5,25 (s, 1H, Protonen in 5-Stellung),

$R_f$ = 0,42 (Benzol/ Dioxan/Essigsäure = 20: 10: 1), 5(E)-2, 3, 4-Trinor-1, 5-inter-m-phenylen-20-methyl-$PGI_2$-Natriumsalz $^1H$-NMR(Deuteromethanol)δ = 0,89 (t, 3H, $CH_3$-), 5,90 (s, 1H, Protonen in 5-Stellung),

$R_f$ = 0,40 (Benzol / Dioxan/ Essigsäure = 20: 10: 1),

5(E, Z)-2, 3, 4-Trinor-1, 5-inter-m-phenylen-20-äthyl-$PGI_2$-Natriumsalz $R_f$ = 0,44 und 0,41 (Benzol / Dioxan / Essigsäure = 20: 10: 1),

2, 3, 4-Trinor-1, 5-inter-m-phenylen-16-amino-$PGI_2$-Natriumsalz $R_f$ = 0,3 (Isopropanol / Wasser = 7: 3),

2, 3, 4-Trinor-1, 5-inter-m-phenylen-16-acetamido-$PGI_2$-Natriumsalz $R_f$ = 0,6 (Isopropanol / Wasser = 7: 3),

2, 3, 4-Trinor-1, 5-inter-m-phenylen-16-benzoylamino-$PGI_2$-Natriumsalz $R_f$ = 0,61 (Isopropanol / Wasser = 7: 3),

2, 3, 4-Trinor-1, 5-inter-m-phenylen-16-tosylamino-$PGI_2$-Natriumsalz $R_f$ = 0,55 (Isopropanol / Wasser = 7: 3),

2, 3, 4, 17, 18, 19, 20-Heptanor-1, 5-inter-m-phenylen-16-amino-16-phenyl-$PGI_2$-Natriumsalz, $R_f$ = 0,36 (Isopropanol / Wasser = 7: 3),

2, 3, 4, 18, 19, 20-Hexanor-1, 5-inter-m-phenylen-16-amino-17-phenyl-$PGI_2$-Natriumsalz ($R_f$ = 0,36, Isopropanol / Wasser = 7: 3).

**Beispiel 10**

5(Z)-2, 3, 4, 17, 18, 19, 20-Heptanor-1, 5-inter-m-phenylen-16-phenoxy-$PGI_2$-Dicyclohexylaminsalz
(Formel I: R1 Dicyclohexylamin $R^2$, $R^3$, $R^4$ = H, $R^5$ = $CH_2-O-Ph$)

399 mg (0,906 mM) 5(Z)-2, 3, 4, 17, 18, 19, 20-Heptanor-1, 5-inter-m-phenylen-16-phenoxy-$PGI_2$-Natriumsalz löst man in 5 ml Wasser, versetzt die Lösung mit 5 ml gesättigter Kochsalzlösung sowie 20 ml Äthylacetat, kühlt das Gemisch auf 0° C ab, stellt seinen pH-Wert unter Rühren mit 1M wäßriger Oxalsäure auf 4 - 5 ein und trennt die Phasen voneinander.

Die wäßrige Phase wird 3 x mit 20 ml Äthylacetat extrahiert. Die vereinigten organischen Phasen werden über Natriumsulfat getrocknet und nach dem Filtrieren von dem Lösungsmittel durch Destillation unter vermindertem Druck befreit. Die als Rückstand erhaltene Säure wird in 2,5 ml Äther gelöst und die Lösung bei Raumtemperatur mit der Lösung von 195 mg (1,08 mM) Dicyclohexylamin in 1 ml Äther versetzt. Dann wird der Äther unter vermindertem Druck abdestilliert, der Rückstand mit Petroläther verrieben und dann dekantiert. Es werden 528 mg (97 %) der Titelverbindung erhalten. $R^f$ 0,43 (Benzol / Dioxan / Essigsäure = 20: 10: 1).

In der gleichen Weise werden die folgenden Verbindungen hergestellt: 5(Z)-2, 3, 4, 17, 18, 19, 20-Heptanor-1, 5-inter-m-phenylen-16-phenoxy-$PGI_2$-Ammoniumsalz, $R_f$ = 0,59 (Benzol/ Dioxan/ Essigsäure = 20: 10: 1),

5(Z)-2, 3, 4, 17, 18, 19, 20-Heptanor-1, 5-inter-m-phenylen-16-phenoxy-$PGI_2$-Dimethylaminsalz, $R_f$ = 0,43 (Benzol/ Dioxan / Essigsäure = 20: l0: l),

5(Z)-2, 3, 4, 17, 18, 19, 20-Heptanor-1, 5-inter-m-phenylen-16-phenoxy-$PGI_2$-Tetrabutylammoniumsalz, $R_f$ = 0,43 (Benzol/ Dioxan / Essigsäure = 20: 10: 1),

5(Z)-2, 3, 4, 17, 18, 19, 20-Heptanor-1, 5-inter-m-phenylen-16-phenoxy-$PGI_2$-Tris-(hydroxymethyl)methylammoniumsalz, $R_f$ = 0,59 (Benzol/ Dioxan / Essigsäure:20: 10: 1).

**Patentansprüche**

1. 2, 3, 4-Trinor-1, 5-inter-m-phenylen-prostacyclin-Derivate der allgemeinen Formel I

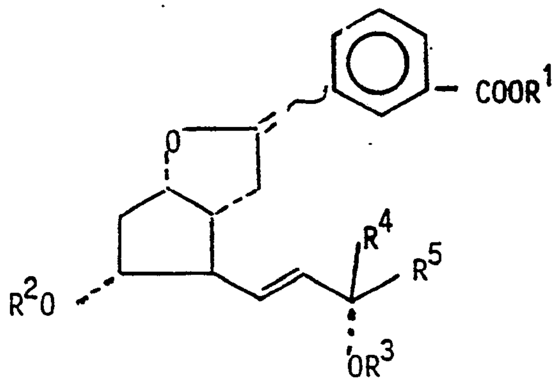

(I) ,

worin

$R^1$ Wasserstoff, $C_{1-4}$-Alkyl, Alkali- oder prim., sec., tert. oder quart. Ammoniumkation,

$R^2$ und $R^3$ unabhängig voneinander Wasserstoff, $C_{1-4}$-Alkanoyl, gegebenenfalls substituiertes Benzoyl oder Äthoxyäthyl,

$R^4$ Wasserstoff oder $C_{1-4}$-Alkyl und

$R^5$ n-Hexyl, n-Heptyl, Phenoxymethyl, m-Trifluormethyl-phenoxymethyl oder eine Gruppe der allgemeinen Formel

$$-\underset{\underset{Z}{|}}{CH}-R^6$$

bedeuten, in der

Z Amino, gegebenenfalls durch Halogen substituiertes $C_{1-4}$-Alkanoylamino, Benzoylamino oder Tosylamino und

$R^6$ $C_{4-6}$-Alkyl, Phenyl oder Benzyl ist.

2. Natriumsalz des 5(Z)-2, 3, 4-Trinor-1, 5-inter-m-phenylen-20-methyl-$PGI_2$,
5(E)-2, 3, 4-Trinor-1, 5-inter-m-phenylen-20-methyl-$PGI_2$ oder des
2, 3, 4-Trinor-1, 5-inter-m-phenylen-20-äthyl-$PGI_2$.

3. Methylester des
2, 3, 4-Trinor-1, 5-inter-m-phenylen-20-methyl-$PGI_2$ oder des
2, 3, 4-Trinor-1, 5-inter-m-phenylen-20-äthyl-$PGI_2$.

4. 2, 3,.4-Trinor-1, 5-inter-m-phenylen-prostacyclin-Derivate der allgemeinen Formel I

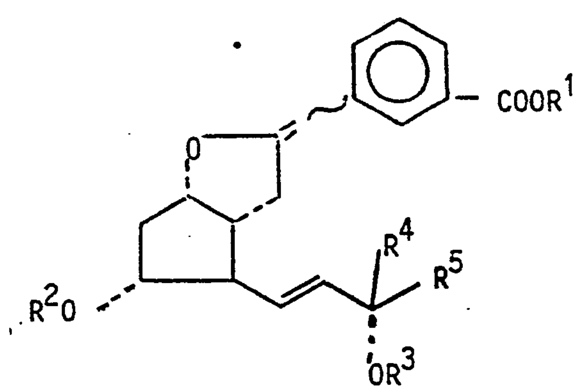

(I) ,

worin

$R^1$, $R^4$, $R^5$, Z und $R^6$ die in Anspruch 1 angegebene Bedeutung haben und

$R^2$ und $R^3$ Tetrahydropyranyl oder Tri-$C_{1-4}$-alkylsilyl bedeuten.

5. Verfahren zur Herstellung von 2, 3, 4-Trinor-1, 5-inter-m-phenylen-prostacyclin-Derivaten der allgemeinen Formel I

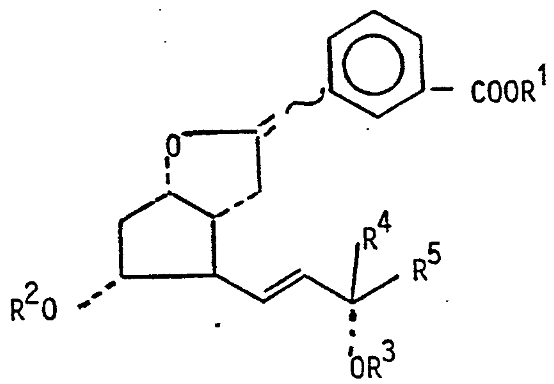

(I) ,

worin

$R^1$ Wasserstoff, $C_{1-4}$-Alkyl, Alkali- oder prim., sec., tert. oder quart. Ammoniumkation,

$R^2$ und $R^3$ unabhängig voneinander Wasserstoff, $C_{1-4}$-Alkanoyl, gegebenenfalls substituiertes Benzoyl oder Äthoxyäthyl,

$R^4$ Wasserstoff oder $C_{1-4}$-Alkyl und

$R^5$ n-Hexyl, n-Heptyl, Phenoxymethyl, m-Trifluormethyl-phenoxymethyl oder eine Gruppe der allgemeinen Formel

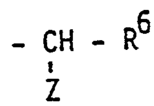

bedeuten, in der

Z Amino, gegebenenfalls durch Halogen substituiertes $C_{1-4}$-Alkanoylamino, Benzoylamino oder Tosylamino und

$R^6$ $C_{4-6}$-Alkyl, Phenyl oder Benzyl ist, dadurch gekennzeichnet,

daß man bicyclische Lactole der allgemeinen Formel II

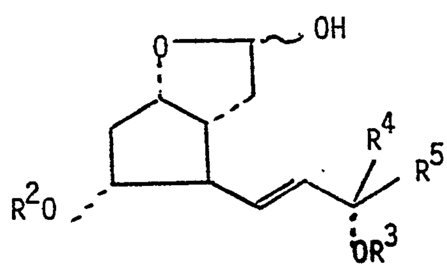

(II) ,

worin

$R^4$, $R^5$, Z und $R^6$ die oben angegebene Bedeutung haben und

$R^2$ und $R^3$ unabhängig voneinander Wasserstoff, $C_{1-4}$-Alkanoyl, gegebenenfalls substituiertes Benzoyl, Äthoxyäthyl, Tetrahydropyranyl oder Tri-$C_{1-4}$-alkylsilyl bedeuten, mit einem Phosphoniumsalz der allgemeinen Formel III

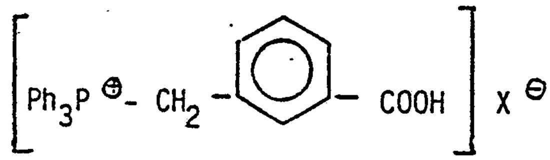 (III)

worin
Ph Phenyl und
X Halogen bedeuten, und einer starken Base in einem dipolaren aprotischen Lösungsmittel umsetzt, das erhaltene Prostaglandin-Derivat der allgemeinen Formel IV

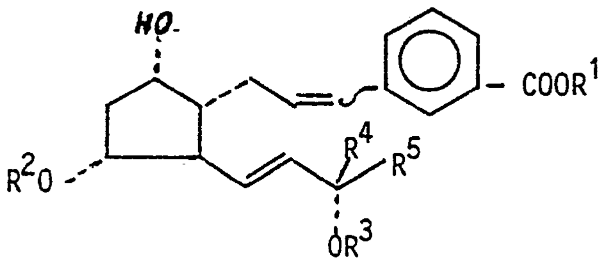 (IV) ,

worin
$R^2$, $R^3$, $R^4$, $R^5$, Z und $R^6$ die oben angegebene Bedeutung haben und
$R^1$ Wasserstoff ist,
gewünschtenfalls nach Überführung in einen Ester, in dem $R^1$ $C_{1-4}$-Alkyl ist, und - sofern der Substituent $R^5$ eine freie Aminogruppe enthält - nach Acylierung mit einem eine gegebenenfalls halogensubstituierte $C_{1-4}$-Alkanoyl-, Benzoyl- oder Tosylgruppe enthaltenden Acylierungsmittel,
durch Behandeln mit einem Halogenierungsmittel der allgemeinen Formel
E - X,
worin
X Chlor, Brom oder Jod und
E Chlor, Brom, Jod, Acetyl, Trifluoracetyl oder den N-Succinimidrest bedeuten,
in einem mit Wasser nicht mischbaren organischen Lösungsmittel, das auch in einem mit Wasser gebildeten Zweiphasensystem vorliegen kann, in An- oder Abwesenheit eines Säureacceptors cyclisiert und aus dem erhaltenen 5-Halogen-$PGI_1$-Derivat der allgemeinen Formel V

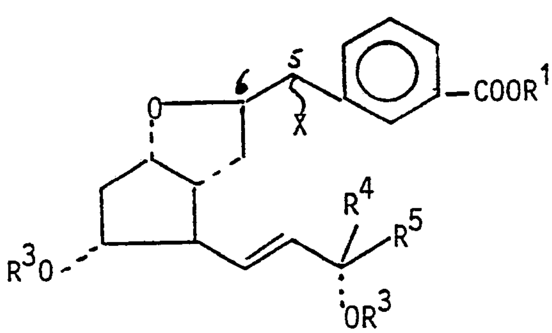 (V) ,

worin
$R^1$, $R^2$, $R^3$, $R^4$, $R^5$, $R^6$ und X die oben angegebene Bedeutung haben
und
Z eine von der freien Aminogruppe verschiedene Bedeutung hat. durch Behandeln mit basischen Reagentien in 5, 6-Stellung Halogenwasserstoff abspaltet, wobei das erhaltene $PGI_2$-Derivat der allgemeinen Formel I

gewünschtenfalls durch Verseifen, Salzbildung, Acylieren oder Desacylieren in ein anderes unter die allgemeine Formel I fallendes $PGI_2$-Derivat übergeführt werden kann.

6. Verfahren nach Anspruch 5, dadurch gekennzeichnet, daß man zur Acylierung der freien Aminogruppe in dem Prostaglandin-Derivat der allgemeinen Formel IV Acetylchlorid, Tosylchlorid, Benzoylchlorid oder Trifluoressigsäure verwendet.

7. Verfahren nach Anspruch 5 oder 6, dadurch gekennzeichnet, daß man als Halogenierungsmittel der allgemeinen Formel E - X elementares Jod oder N-Bromsuccinimid verwendet.

8. Verfahren nach Anspruch 5, 6 oder 7, dadurch gekennzeichnet, daß man die Umsetzung mit dem Halogenierungsmittel in Gegenwart von Natriumhydrogencarbonat als Säureacceptor durchführt.

9. Verfahren nach Anspruch 5, 6, 7 oder 8 dadurch gekennzeichnet, daß man zur Halogenwasserstoffabspaltung aus dem 5-Halogen-$PGI_1$-Derivat der allgemeinen Formel V 1, 5-Diazabicyclo[4, 3, 0]non-5-en verwendet.

10. Pharmazeutische Präparate, die als Wirkstoff eine oder mehrere der 2, 3, 4-Trinor-1, 5-inter-m-phenylen-prostacyclin-Derivate der Ansprüche 1 bis 3 enthalten.

**Claims**

1. 2, 3, 4-trinor-1, 5-inter-m-phenylene-prostacyclin-derivatives of general formula I

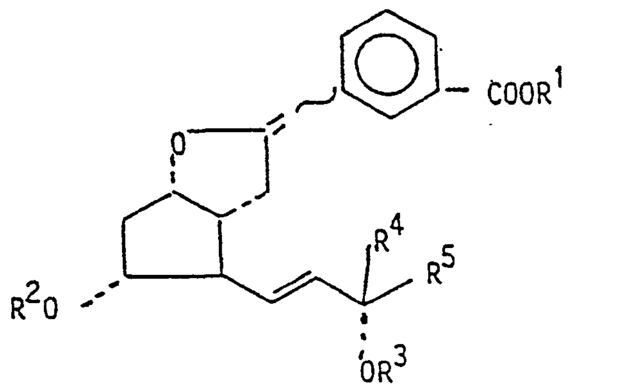

(I) ,

in which

$R^1$ stands for hydrogen, $C_{1-4}$-alkyl, alkali or prim., sec., tert. or quart. ammonium cation,

$R^2$ and $R^3$, independently of one another, hydrogen, $C_{1-4}$-alkanoyl, optionally substituted benzoyl or ethoxyethyl,

$R^4$ hydrogen or $C_{1-4}$-alkyl and

$R^5$ n-hexyl, n-heptyl, phenoxymethyl, m-trifluoromethylphenoxymethyl or a group of general formula

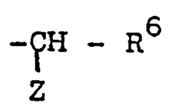

in which

Z stands for amino, optionally halogen-substituted $C_{1-4}$-alkanoylamino, benzoylamino or tosylamino and

$R^6$ $C_{4-6}$-alkyl, phenyl or benzyl.

2. The sodium salt of 5(Z)-2, 3, 4-trinor-1, 5-inter-m-phenylene-20-methyl-$PGI_2$,
5(E)-2, 3, 4-trinor-1, 5-inter-m-Phenylene-20-methyl-$PGI_2$ or 2, 3, 4-trinor-1, 5-inter-m-phenylene-20-ethyl-$PGI_2$.

3. The methyl ester of 2, 3, 4-trinor-1, 5-inter-m-phenylene-20-methyl-$PGI_2$ or 2, 3, 4-trinor-1, 5-inter-m-phenylene-20-ethyl-$PGI_2$.

4. 2, 3, 4-trinor-1, 5-inter-m-phenylene-prostacyclin derivatives of general formula I

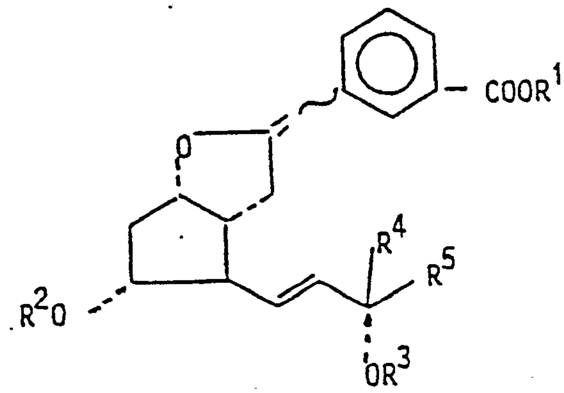

(I) ,

in which $R^1$, $R^4$, $R^5$, Z and R have the meanings given in claim 1 and $R^2$ and $R^3$ stand for tetrahydropyranyl or tri-$C_{1-4}$-alkyl silyl.

5. Process for the preparation of 2, 3, 4-trinor-1, 5-inter-m-phenylene-prostacyclin derivatives of general formula I

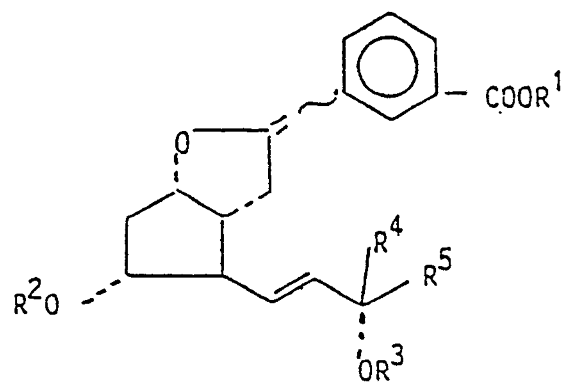

(I) ,

in which

$R^1$ stands for hydrogen, $C_{1-4}$-alkyl, alkali or prim., sec., tert. or quart. ammonium cation,

$R^2$ and $R^3$, independently of one another, hydrogen, $C_{1-4}$-alkanoyl, optionally substituted benzoyl or ethoxyethyl,

$R^4$ hydrogen or $C_{1-4}$-alkyl and

$R^5$ n-hexyl, n-heptyl, phenoxymethyl, m-trifluoromethylphenoxymethyl or a group of general formula

$$\begin{array}{l}-\underset{\displaystyle Z}{\underset{|}{CH}} - R^6\end{array}$$

in which

Z stands for amino, optionally halogen substituted $C_{1-4}$-alkanoylamino, benzoylamino or tosylamino and

$R^6$ $C_{4-6}$-alkyl, phenyl or benzyl, characterized in that bicyclic lactols of general formula II

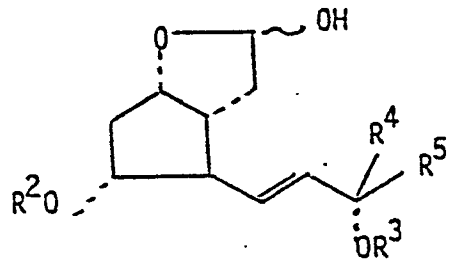

(II) ,

in which $R^4$, $R^5$, Z and $R^6$ have the aforementioned meaning, and $R^2$ and $R^3$, independently of one another, stand for hydrogen, $C_{1-4}$-alkanoyl, optionally substituted benzoyl, ethoxyethyl, tetrahydropyranyl or tri-$C_{1-4}$-alkyl silyl are reacted with a phosphonium salt of general formula III

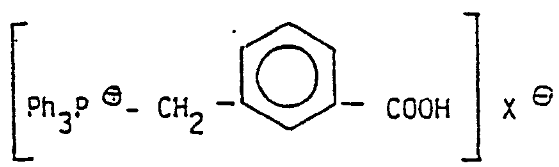

(III)

in which Ph stands for phenyl and X for halogen, and a strong base in a dipolar aprotic solvent, the obtained prostaglandin derivative of general formular IV

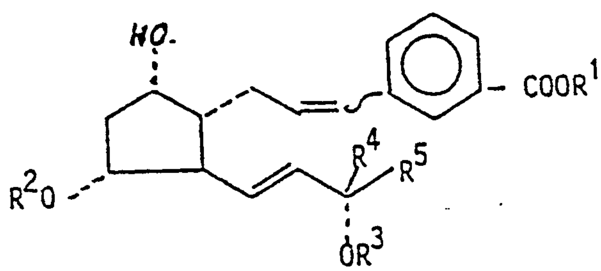

(IV) ,

in which $R^2$, $R^3$, $R^4$, $R^5$, Z and $R^6$ have the aforementioned meanings and $R^1$ is hydrogen, optionally after converting into an ester, in which $R^1$ is $C_{1-4}$-alkyl and - if the substituent $R^5$ contains a free amino group - after acylation with an acylating agent optionally containing a halogen-substituted $C_{1-4}$-alkanoyl, benzoyl or tosyl group is cyclized by treatment with a halogenating agent of general formula E - X, in which X stands for chlorine, bromine or iodine and E for chlorine, bromine, iodine, acetyl, trifluoroacetyl or the N-succinimide radical in a water-immiscible organic solvent, which can also be present in a two-phase system formed with water, in the presence or absence of an acid acceptor and hydrogen halide is split off from the 5-halogen-$PGI_1$ derivative obtained of general formula V

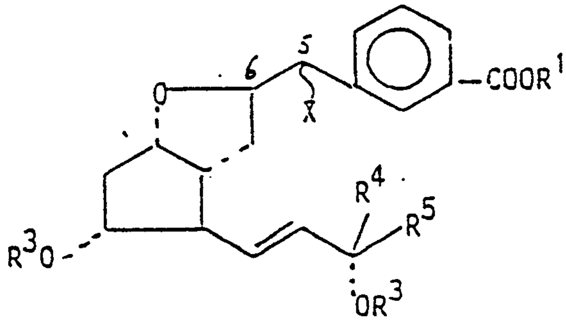

(V) ,

in which $R^1$, $R^2$, $R^3$, $R^4$, $R^5$, $R^6$ and X have the aforementioned meanings and Z has a meaning different from the free amino group, by treating with basic reagents in the 5,6-position, the PGI derivative of general formula I obtained being optionally convertable by saponification, salification, acylation or deacylation into another $PGI_2$ derivative covered by the general formula I.

6. Process according to claim 5, characterized in that acetyl chloride, tosyl chloride, benzoyl chloride or trifluoroacetic acid is used for acylating the free amino group in the prostaglandin derivative of general formula IV.

7. Process according to claims 5 or 6, characterized in that elementary iodine or N-bromosuccinimide is used as the halogenating agent of general formula E - X.

8. Process according to claims 5, 6 or 7, characterized in that the reaction is carried out by the halogenating agent in the presence of sodium hydrogen carbonate as the acid acceptor.

9. Process according to claims 5, 6, 7 or 8, characterized in that 1,5-diazabicyclo[4, 3, 0]non-5-ene is used for the splitting off of hydrogen halide from the 5-halogen-$PGI_1$ derivative of general formula V.

10. Pharmaceutical products containing as the active substance one or more of the 2, 3, 4-trinor-1, 5-inter-m-phenylene prostacyclin derivatives of claims 1 to 3.

## Revendications

1 - Dérivés de la 2, 3, 4-trinor-1, 5-inter-m-phénylène-prostacycline de formule générale

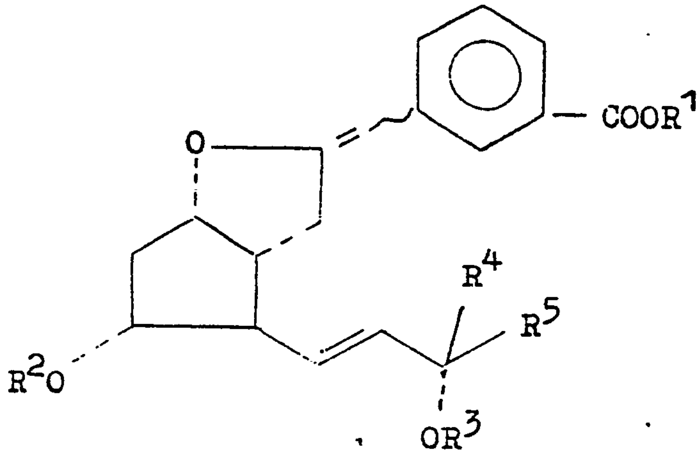

(I),

dans laquelle

$R^1$ représente l'hydrogène, un groupe alkyle en C 1-C 4, un cation alcalin ou un cation d'ammonium primaire, secondaire, tertiaire ou quaternaire,

$R^2$ et $R^3$ représentent chacun, indépendamment l'un de l'autre, l'hydrogène, un groupe alcanoyle en C 1-C 4, benzoyle éventuellement substitué ou éthoxyéthyle,

$R^4$ représente l'hydrogène ou un groupe alkyle en C 1-C 4, et

$R^5$ représente un groupe n-hexyle, n-heptyle, phénoxyméthyle, m-trifluorométhyl-phénoxyméthyle ou un groupe de formule générale

$- CH - R^6$
$\quad\ \ Z$

dans laquelle

Z représente un groupe amino, alcanoylamino en C 1-C 4, benzoylamino ou toluène-sulfonylamino éventuellement substitué par des halogènes, et

$R^6$ représente un groupe alkyle en C 4-C 6, phényle ou benzyle.

2 - Le sel de sodium de
la 5(Z)-2, 3, 4-trinor-1, 5-inter-m-phénylène-20-méthyl-$PGI_2$,
la 5(E)-2, 3, 4-trinor-1, 5-inter-m-phénylène-20-méthyl-$PGI_2$ ou de
la 2, 3, 4-trinor-1, 5-inter-m-phénylène-20-éthyl-$PGI_2$.

3 - L'ester méthylique de
la 2, 3, 4-trinor-1, 5-inter-m-phénylène-20-méthyl-$PGI_2$ ou de
la 2, 3, 4-trinor-1, 5-inter-m-phénylène-20-éthyl-$PGI_2$.

4 - Dérivés de la 2,3,4-trinor-1,5-inter-m-phénylène-prostacycline de formule générale I

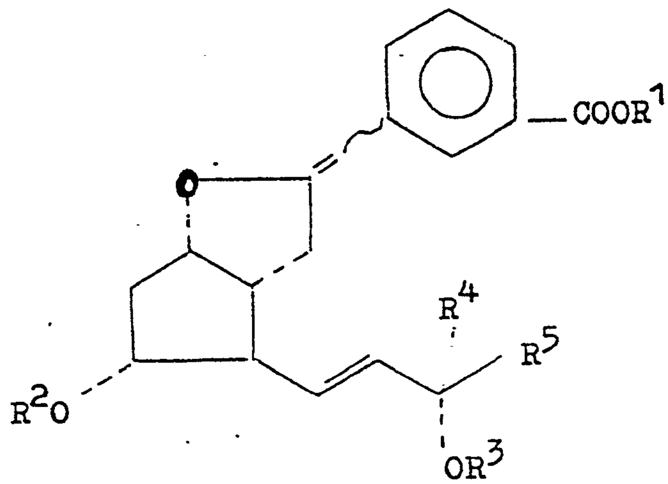

(I),

dans laquelle

$R^1$, $R^4$, $R^5$, Z et $R^6$ ont les significations indiquées dans la revendication 1, et

$R^2$ et $R^3$ représentent des groupes tétrahydropyrannyle ou tri-(alkyle en C 1-C 4)-silyle.

5 - Procédé de préparation des dérivés de la
2, 3, 4-trinor-1, 5-inter-m-phénylène-prostacycline de formule gènérale I

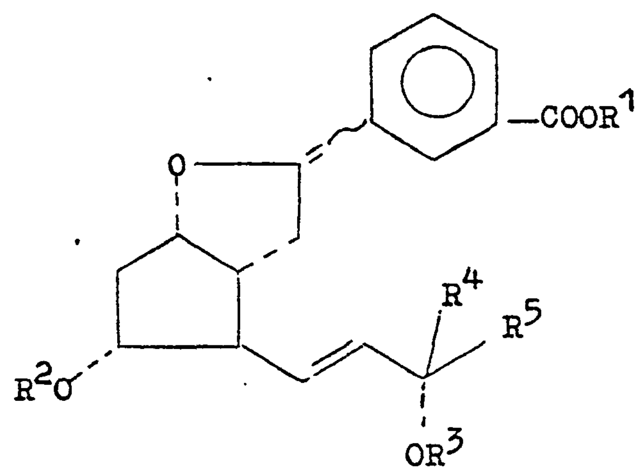

(I),

dans laquelle

$R^1$ représente l'hydrogène, un groupe alkyle en C 1-C 4, un cation alcalin ou d'ammonium primaire, secondaire, tertiaire ou quaternaire,

$R^2$ et $R^3$ représentent chacun, indépendamment l'un de l'autre, l'hydrogène, un groupe alcanoyle en C 1-C 4,

benzoyle éventuellement substitué ou éthoxyéthyle,

$R^4$ représente l'hydrogène ou un groupe alkyle en C 1-C 4, et

$R^5$ représente un groupe n-hexyle, n-heptyle, phénoxyméthyle, m-trifluorométhyl-phénoxyméthyle ou un groupe de formule générale

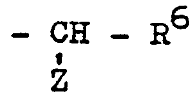

dans laquelle

Z représente un groupe amino, un groupe alcsnoylamino en C 1-C 4, benzoylamino ou toluène-sulfonylamino éventuellement substitué par des halogènes, et

$R^6$ représente un groupe alkyle en C 4-C 6, phényle ou benzyle,

caractérisé en ce que l'on fait réagir des lactols bicycliques de formule générale II

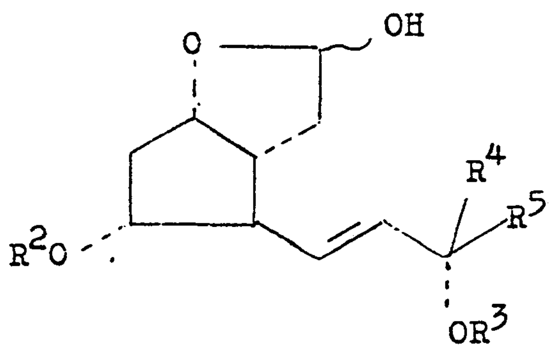

(II),

dans laquelle

$R^4$, $R^5$, Z et $R^6$ ont les significations indiquées ci-dessus, et

$R^2$ et $R^3$ reprèsentent chacun, indépendamment l'un de l'autre, l'hydrogène, un groupe alcanoyle en C 1-C 4, un groupe benzoyle éventuellement substitué, éthoxyéthyle, tétrahydropyrannyle ou tri-(alkyle en C 1-C 4)-silyle,

avec un sel de phosphonium de formule générale III

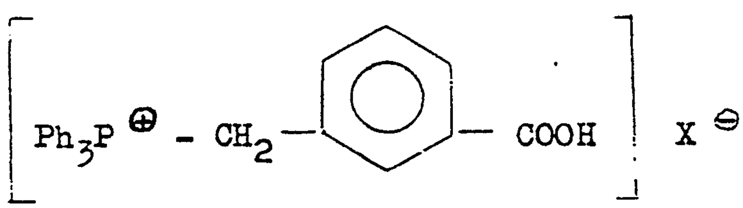

(III)

dans laquelle

Ph représente un groupe phényle, et

X un halogène,

et une base forte dans un solvant aprotonique dipolaire, ce qui donne un dérivé de prostaglandine de formule générale IV

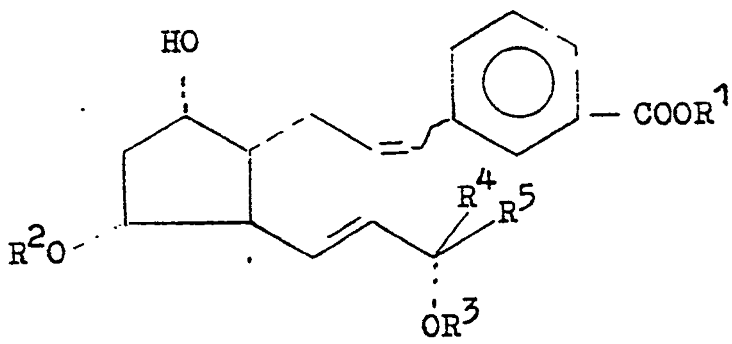

(IV),

dans laquelle $R^2$, $R^3$, $R^4$, $R^5$, Z, et $R^6$ ont les significations indiquées ci-dessus, et

$R^1$ représente l'hydrogène,

qu'on cyclise, éventuellement après conversion en un ester dans lequel $R^1$ représente un groupe alkyle en C 1-C 4 et - lorsque le substituant $R^5$ contient un groupe amino libre - après acylation à l'aide d'un agent acylant contenant un groupe alcanoyle en C 1-C 4, benzoyle ou toluène-sulfonyle éventuellement substitué, par traitement à l'aide d'un agent halogénant de formule générale

E - X

dans laquelle

X représente le chlore, le brome ou l'iode, et

E représente le chlore le brome, l'iode, un groupe acétyle, trifluoracétyle ou le reste du N-succinimide,

dans un solvant organique non miscible à l'eau qui peut également se trouver dans un système à deux phases formé avec de l'eau, en présence ou en l'absence d'un accepteur d'acide, ce qui donne le dérivé de 5-halogéno-$PGI_1$ de formule générale V

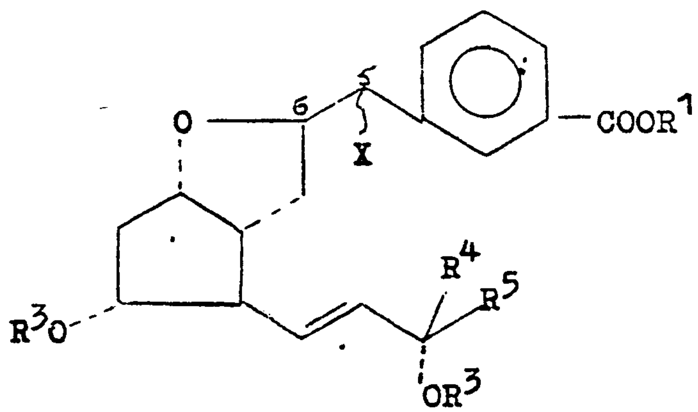

(V),

dans laquelle R 1, $R^2$, $R^3$, $R^4$, $R^5$, $R^6$ et X ont les significations indiquées ci-dessus et

Z a une signification autre qu'un groupe amino libre,

d'où l'on élimine, par traitement à l'aide de réactifs basiques, un halogénure d'hydrogène de la position 5,6, ce qui donne un dérivé de $PGI_2$ de formule générale I qu'on peut convertir si on le désire par saponification, salification, acylation ou désacylation en un autre derivé de $PGI_2$ de formule générale I.

6 - Procédé selon la revendication 5, caractérisé en ce que pour l'acylation du groupe amino libre dans le dérivé de prostaglandine de formule générale IV, on utilise le chlorure d'acétyle, le chlorure de toluène-sulfonyle, le chlorure de benzoyle ou l'acide trifluoracétique.

7 - Procédé selon la revendication 5 ou 6, caractérisé en ce que l'on utilise en tant qu'agent halogénant de formule générale E-X l'iode élémentaire ou le N-bromosuccinimide.

8 - Procédé selon les revendications 5, 6 ou 7, caractérisé en ce que la réaction avec l'agent halogenant est effectuée en présence de bicarbonate de sodium qui sert d'accepteur d'acide.

9 - Procédé selon la revendication 5, 6, 7 ou 8, caractérisé en ce que pour éliminer un halogénure d'hydrogène du dérivé de 5-halogéno-$PGI_1$ de formule generale V, on utilise le 1,5-diazabicyclo[4,3,0]nona-5-ène.

10 - Compositions pharmaceutiques contenant en tant que substsnce active un ou plusieurs dérivés de la 2, 3, 4-trinor-1, 5-inter-m-phénylène-prostacycline selon les revendications 1 à 3.